(19) Europäisches Patentamt
European Patent Office
Office européen des brevets

(11) Veröffentlichungsnummer: 0 010 516
B1

(12) # EUROPÄISCHE PATENTSCHRIFT

(45) Veröffentlichungstag der Patentschrift: 14.07.82

(21) Anmeldenummer: 79810102.8

(22) Anmeldetag: 21.09.79

(51) Int. Cl.³: C 08 K 5/34, C 07 D 211/46, C 07 D 471/10, C 07 D 491/10, C 07 D 491/22, C 07 D 498/10

(54) Neue Polyalkylpiperidinderivate, ihre Verwendung als Lichtschutzmittel und damit stabilisierte Polymere.

(30) Priorität: 27.09.78 CH 10088/78

(43) Veröffentlichungstag der Anmeldung: 30.04.80 Patentblatt 80/9

(45) Bekanntmachung des Hinweises auf die Patenterteilung: 14.07.82 Patentblatt 82/28

(84) Benannte Vertragsstaaten: BE CH DE FR GB IT NL

(56) Entgegenhaltungen:
US-A-4 118 368
PATENT ABSTRACTS OF JAPAN, Vol. 1, Nr. 61, 15. Juni 1977, Seite 836 C 77. Tokyo, (JP)

(73) Patentinhaber: CIBA-GEIGY AG, Patentabteilung Postfach, CH-4002 Basel (CH)

(72) Erfinder: Rody, Jean, Dr., Rütiring 82, D-4125 Riehen (CH)
Erfinder: Slongo, Mario, Dr., Unterwartweg 27, D-4132 Muttenz (CH)

EP 0 010 516 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Neue Polyalkylpiperidinderivate, ihre Verwendung als Lichtschutzmittel und damit stabilisierte Polymere

Die Erfindung betrifft neue Lichtschutzmittel für organische Materialien, die durch UV-Licht geschädigt werden, insbesondere für organische Polymere. Diese Lichtschutzmittel stellen molekulare Kombinationen von Benztriazolen oder 2-Hydroxybenzophenonen und Polyalkylpiperidinderivaten dar.

Es ist bekannt, daß 2-(2'-Hydroxyphenyl)-benztriazole und 2-Hydroxybenzophenone wertvolle Lichtschutzmittel für organische Materialien sind. Sie wirken als UV-Absorber, das heißt, sie wandeln Lichtquanten hoher Energie (UV-Licht) in Wärme um, die für das zu schützende organische Material ohne nachteilige Wirkung ist.

Es ist weiterhin bekannt, daß Polyalkylpiperidine ebenfalls wertvolle Lichtschutzmittel sind, die jedoch nicht als UV-Absorber wirken, da sie im kurzwelligen Licht nicht absorbieren. Der Wirkungsmechanismus konnte bisher noch nicht eindeutig geklärt werden, siehe z. B.: H. J. Heller und H. R. Blattmann, Pure and Applied Chemistry, 36 (1973), 141–161.

Überraschend wurde gefunden, daß molekulare Kombinationen von Benztriazolen oder Hydroxybenzophenonen einerseits und Polyalkylpiperidinen andererseits eine Lichtschutzwirkung besitzen, die weit höher ist, als sich aus der Summe der beiden Komponenten erwarten läßt. Ein solcher Synergismus war nicht vorherzusehen.

Gegenstand der Erfindung sind daher Verbindungen, die in ihrem Molekül sowohl mindestens eine 2-(2'-Hydroxyphenyl)-benztriazol-Gruppe oder eine 2-Hydroxybenzophenon-Gruppe als auch mindestens eine Polyalkylpiperidingruppe enthalten, insbesondere Verbindungen, die eine oder zwei Gruppen der Formel AI oder AII

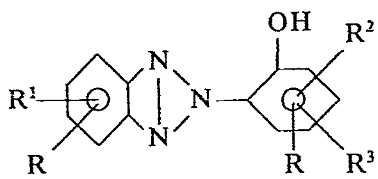

(A I)

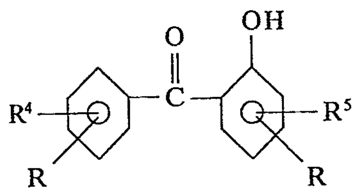

(A II)

und eine oder zwei der Gruppen B I bis B XIII enthalten,

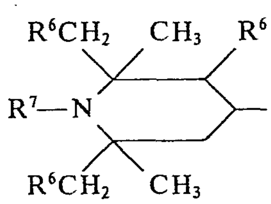

(BI)

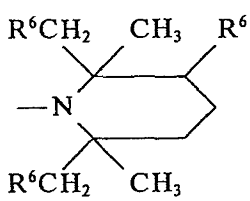

(BII)

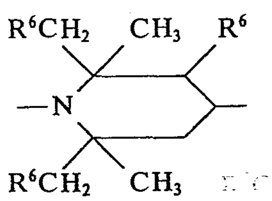

(BIII)

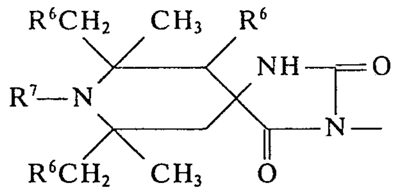

(B IV)

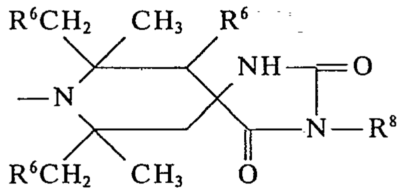

(B V)

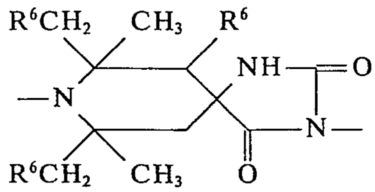

(B VI)

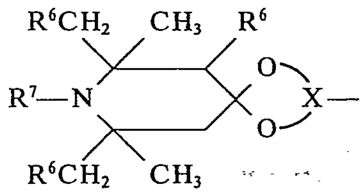

(B VII)

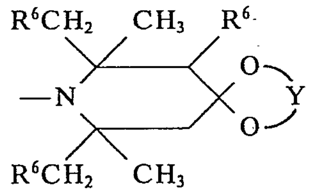

(B VIII)

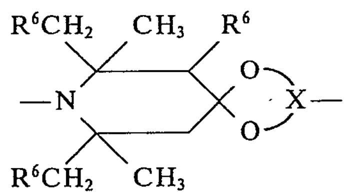

(B IX)

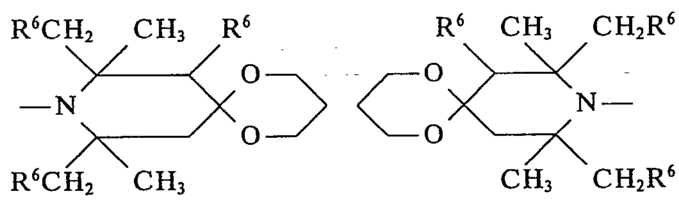

(B X)

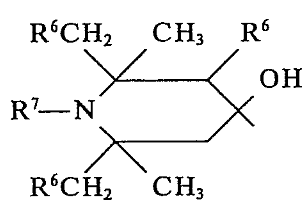

(B XI)

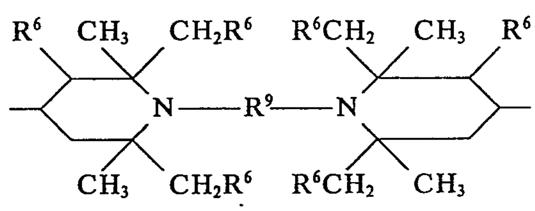

(B XII)

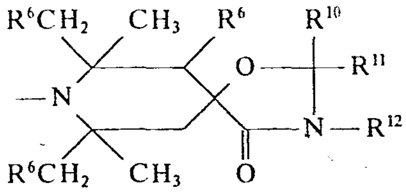

(BXIII)

worin R Wasserstoff oder eine freie Valenz,

$R^1$ Wasserstoff, Chlor, $C_1$–$C_4$-Alkyl, OH oder $C_1$–$C_4$-Alkoxy,

$R^2$ und $R^3$ unabhängig voneinander H, Cl, $C_1$–$C_{12}$-Alkyl, $C_5$–$C_8$-Cycloalkyl, Phenyl, $C_7$–$C_9$-Phenylalkyl, OH oder $C_1$–$C_{12}$-Alkoxy,

$R^4$ und $R^5$ unabhängig voneinander H, Cl, OH, $C_1$–$C_{12}$-Alkoxy oder $C_1$–$C_{12}$-Alkyl,

$R^6$ H oder $CH_3$,

$R^7$ H, O˙, $C_1$–$C_{12}$-Alkyl, $C_3$–$C_8$-Alkenyl, Benzyl, Acetyl oder eine Gruppe

$$-CH_2-CH(OH)-R^{13}$$

$R^8$ $C_1$–$C_{12}$-Alkyl, $C_3$–$C_8$-Alkenyl oder $C_7$–$C_9$-Phenylalkyl,

$R^9$ eine Gruppe

$-CH_2CH{=}CHCH_2$ 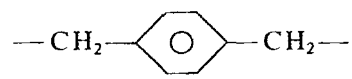

oder

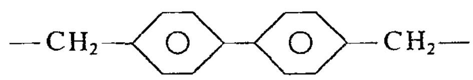

$R^{10}$ und $R^{11}$ Wasserstoff, $C_1$–$C_{12}$-Alkyl, Phenyl oder Benzyl, oder

$R^{10}$ und $R^{11}$ zusammen $C_4$–$C_{19}$-Alkylen,

$R^{12}$ H oder eine freie Valenz,

$R^{13}$ H, $CH_3$, $C_2H_5$ oder Phenyl,

X eine Gruppe

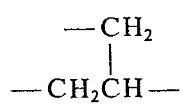 oder 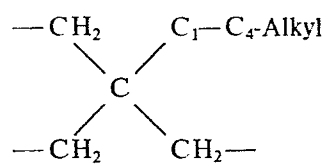

und

Y eine $C_2$–$C_8$-Alkylengruppe bedeuten,

wobei die Gruppen A mit den Gruppen B durch eine direkte Bindung oder durch ein zweiwertiges Zwischenglied –Z– verbunden sind und –Z– eine der folgenden Gruppen sein kann:

$-(CH_2)_m-CO-O-$ $\quad -(CH_2)_m-CO-O-R^{14}-$ $\quad -O-(CH_2)_n-CO-O-R^{14}-$

$-CH_2-CH(OH)-CH_2-O-CO-(CH_2)_m-$

$-O-CH_2-CH(OH)-CH_2-O-CO-(CH_2)_m-$

$-CH_2-CH(OH)-CH_2-$ $\quad -CH_2-CH(OH)-CH_2-O-$

$-CH_2-CH(OH)-CH_2-N(R^{15})-(CH_2)_m-$

$-O-CH_2-CH(OH)-CH_2-N(R^{15})-(CH_2)_m-$

$-N(R^{15})-CH_2-CH(OH)-CH_2-N(R^{16})-(CH_2)_m-$

$-CH_2-CH(R^{13})-O-CH_2-CH(OH)-CH_2-N(R^{15})-(CH_2)_m-$

$-CH_2-CH(R^{13})-O-CH_2-CH(OH)-CH_2-O-CO-(CH_2)_m-$

$-(CH_2)_m-N(R^{15})-CO-$ $\quad -(CH_2)_m-N(R^{15})-CO-(CH_2)_n-$

$-O-(CH_2)_3-N(R^{15})-CO-(CH_2)_m-$ $\quad -CH_2-NH-CO-CH_2-O-$

$-CH_2-NH-CO-CH_2-O-CH(R^{13})-CH_2-$

$—CH_2—NH—CO—CH_2—N(R^{15})—(CH_2)_m—$

$—CH_2—NH—CO—CH_2CH_2—N(R^{15})—(CH_2)_m—$

oder

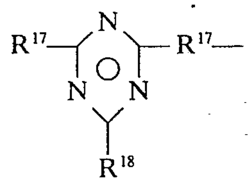

worin m 0, 1 oder 2 ist, n 1 oder 2 ist, $R^{14}$ $-CH_2-$ oder $-CH(R^{13})-CH_2-$ bedeutet, $R^{15}$ und $R^{16}$ H, $C_1-C_{12}$-Alkyl, Cyclohexyl oder Benzyl bedeuten, $R^{17}$ $-O-$ oder $-NR^{15}-$ darstellt und $R^{18}$ $C_1-C_{12}$-Alkoxy, Phenoxy oder eine Aminogruppe

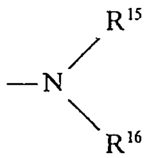

bedeutet, oder die Gruppen A und B durch einen dreiwertigen Rest der Formel

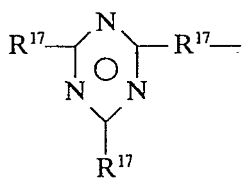

verbunden sind, oder eine Gruppe A II mit einer Gruppe B über einen Rest $-O-(CH_2)_n-CO-O-$ verbunden ist.

Wenn die Gruppen A mit den Gruppen B über zweiwertige Zwischenglieder Z verbunden sind, so sind die folgenden Strukturen möglich:

A—Z—B B—Z—A—Z—B A—Z—B—Z—A

Analoge Strukturen sind möglich, wenn A und B durch direkte Bindungen verbunden sind. Wenn A mit B über einen dreiwertigen Triazinrest T verbunden sind, so sind die folgenden Strukturen möglich:

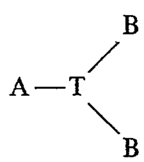

oder

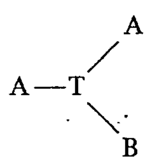

$R^1$ als Alkyl kann hierbei z. B. Methyl, Äthyl, Isopropyl oder tert.Butyl sein. $R^2$, $R^3$, $R^4$, $R^7$, $R^8$, $R^{10}$, $R^{11}$, $R^{15}$ und $R^{16}$ als Alkyl können darüber hinaus auch z. B. n-Hexyl, 2-Äthylhexyl, Isononyl, n-Decyl oder n-Dodecyl sein.

$R^2$ und $R^3$ als Cycloalkyl können beispielsweise Cyclopentyl, Cyclohexyl, Dimethylcyclohexyl oder Cyclooctyl sein.

$R^7$ und $R^8$ als $C_3-C_8$-Alkenyl können z. B. Allyl, Methallyl, 2-Hexenyl oder 2-Octenyl sein.

$R^2$, $R^3$ und $R^8$ als $C_7-C_9$-Phenyl können z. B. Benzyl, Phenyläthyl oder Phenylpropyl sein.

$R^1$ als $C_1-C_4$-Alkoxy kann beispielsweise Methoxy, Äthoxy, Isopropoxy oder tert.Butoxy sein. $R^2$, $R^3$, $R^4$, $R^5$ und $R^{18}$ als $C_1-C_{12}$-Alkoxy können darüber hinaus auch z. B. Hexyloxy, Octyloxy, Nonyloxy oder Dodecyloxy sein.

Y als $C_2-C_8$-Alkylen kann beispielsweise 1,2-Äthylen, 1,2-Propylen, 1,3-Propylen, 1,2-Butylen, 2,3-Butylen, 2,2-Dimethyl-1,3-propylen, 2-Äthyl-1,3-propylen oder 1,2-Octylen sein. In den Gruppen der Formel B VIII bildet Y zusammen mit den beiden Sauerstoffatomen und dem tertiären C-Atom einen 5- oder 6gliedrigen Ring.

Wenn $R^{10}$ und $R^{11}$ zusammen $C_4-C_{19}$-Alkylen sind, so bilden sie zusammen mit dem tertiären C-Atom, an das sie gebunden sind, einen $C_5$-$C_{20}$-Cycloalkan-Ring, beispielsweise einen Cyclopentan-, Cyclohexan-, Methylcyclohexan-, Cyclooctan, Cyclododecan oder Cycloeikosan-Ring.

Bevorzugt ist $R^1$ Wasserstoff oder Chlor. Bevorzugt sind unter den B-Gruppen die Gruppen B I bis B VI und darin solche Gruppen, in denen $R^6$ Wasserstoff, $R^7$ Wasserstoff, $C_1-C_8$ Alkyl, Allyl oder Benzyl und $R^8$ $C_1-C_{12}$-Alkyl, $C_3-C_5$-Alkenyl oder Benzyl ist.

Unter den Bindegliedern Z sind bevorzugt die Gruppen

$-(CH_2)_m-CO-O-$, $-(CH_2)_m-CO-O-R^{14}-$, $-O-(CH_2)_n-CO-O-$, $-O-(CH_2)_n-CO-O-R^{14}-$, $-(CH_2)_m-N(R^{15})-CO-$, $-(CH_2)_m-N(R^{15})-CO-(CH_2)_n-$, $-CH_2-NH-CO-CH_2-O-$ und $-CH_2-NH-CO-CH_2-N(R^{15})-(CH_2)_m-$.

Unter den Triazin-Bindegliedern sind solche bevorzugt worin $R^{17}$ eine Gruppe $-NR^{15}-$ ist.

Beispiele für erfindungsgemäße Verbindungen sind die Verbindungen der folgenden Formeln:

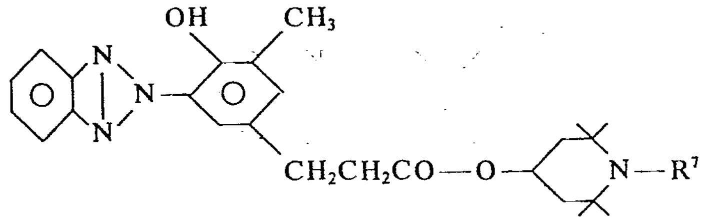

($R^7$ = H, $CH_3$, Benzyl oder Acetyl)

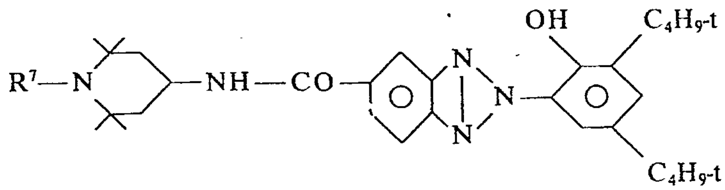

($R^7$ = H, O, Butyl oder Allyl)

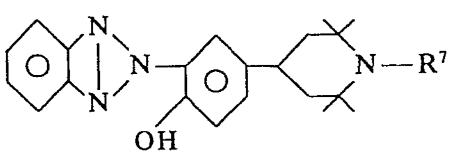

($R^7$ = H oder $CH_3$)

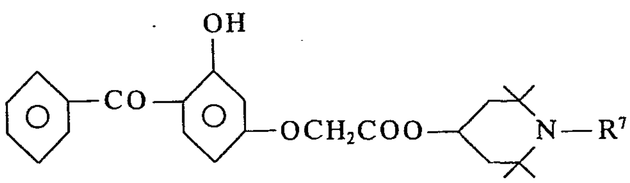

($R^7$ = H oder $CH_3$)

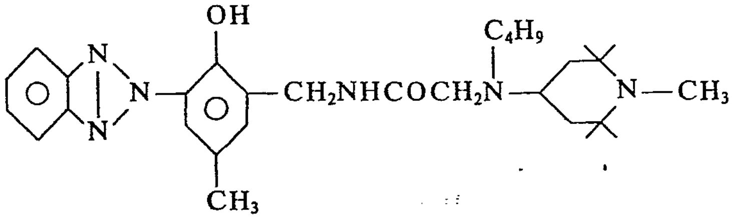

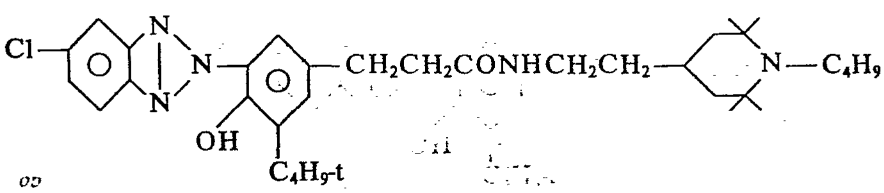

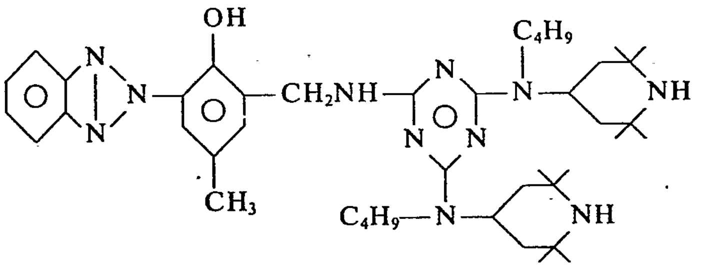

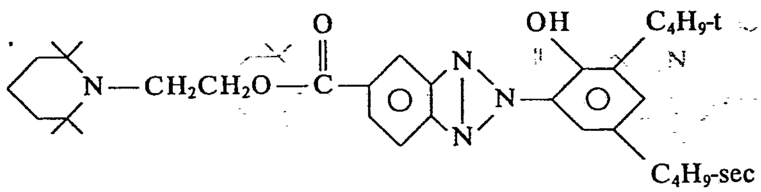

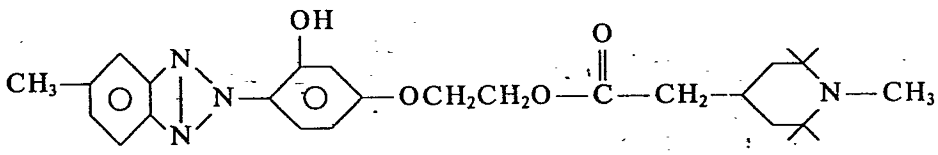

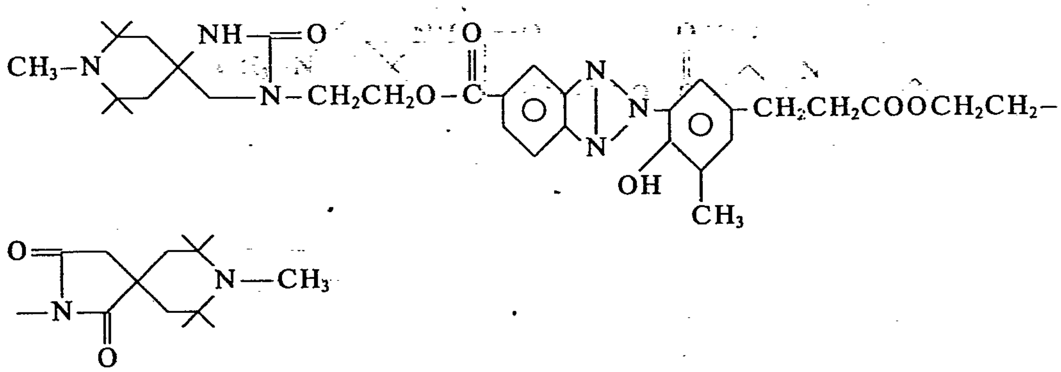

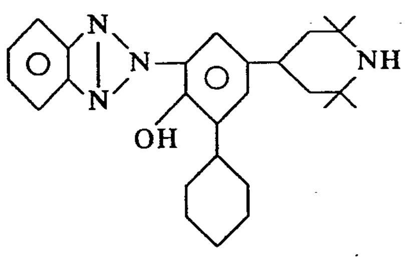

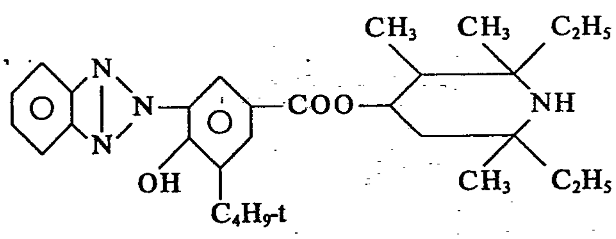

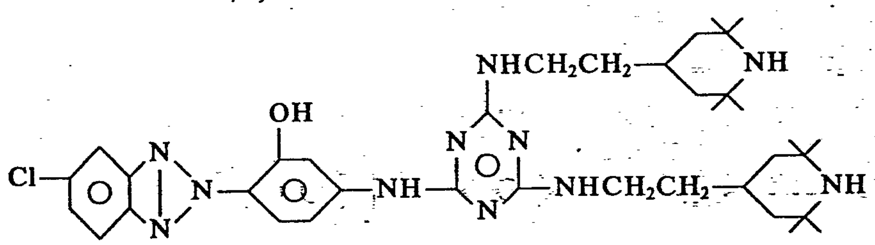

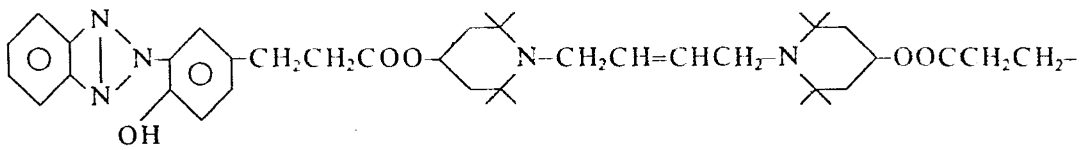

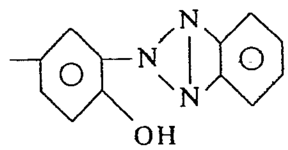

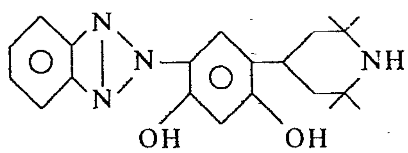

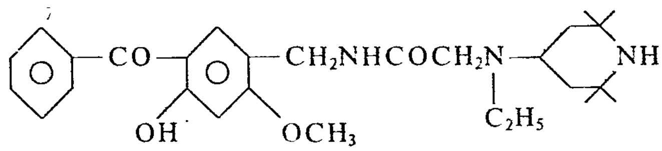

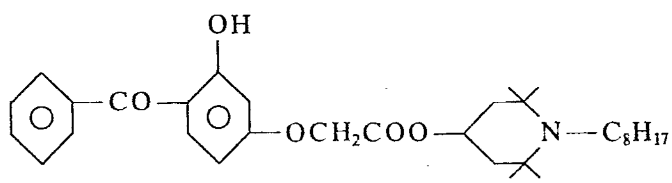

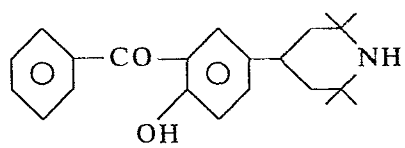

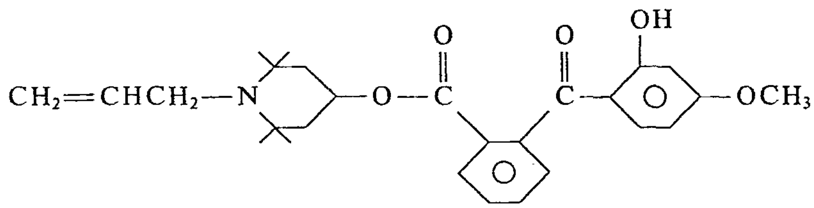

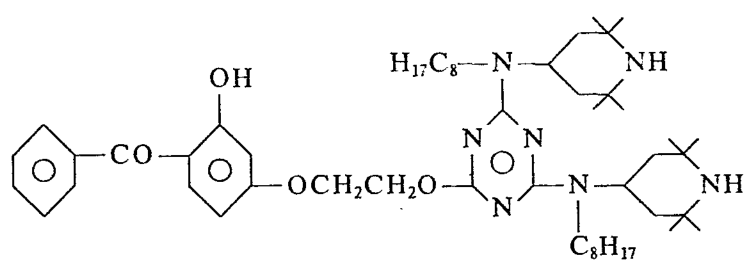

Zur Herstellung der erfindungsgemäßen Verbindungen geht man von einer Verbindung der Formel AI oder AII aus, in der mindestens ein R eine funktionelle Gruppe ist, die mit einem funktionellen Polyalkylpiperidin-Derivat reagieren kann.

Ist die Gruppe R beispielsweise eine Carbonsäuregruppe $-COOH$, $-CH_2CH_2COOH$ oder $-OCH_2COOH$ oder deren Niederalkylester oder deren Säurechlorid, so läßt sich eine solche Verbindung AI oder AII mit einer Polyalkylpiperidinverbindung umsetzen, die eine funktionelle Hydroxy- oder Aminogruppe trägt, z. B. $-OH$, $-CH_2CH_2OH$, $-NH_2$, $-CH_2NH_2$ oder $-CH_2CH_2NH_2$.

Ist die Gruppe R eine Aminogruppe, z. B. $-NH_2$, $-CH_2NH_2$ oder $-CH_2CH_2NH_2$, so läßt sich eine solche Verbindung AI oder AII mit einer Polyalkylpiperidinverbindung umsetzen, die eine

Carboxylgruppe trägt oder ein Derivat davon wie z. B. eine Alkoxycarbonyl- oder Chlorcarbonylgruppe. In beiden Fällen entstehen Ester oder Amide und es wird nach den üblichen Methoden der Veresterung oder Amidierung gearbeitet.

Trägt eine der beiden Komponenten eine Epoxyalkylgruppen und die andere Komponente eine OH- oder NH-Gruppe, so entstehen Zwischenglieder mit Äther- oder Amingruppen.

Solche Verbindungen, in denen das Zwischenglied ein Triazinrest ist, werden durch stufenweise Reaktion eines Dichlortriazins oder von Cyanursäurechlorid mit

a) einem OH- oder NH-Derivat von AI oder AII und
b) einem OH- oder NH-Derivat eines Polyalkylpiperidins hergestellt.

Bei der Reaktion von Cyanursäurechlorid kann man dieses entweder mit 2 Äquivalenten A-Derivat und 1 Äquivalent B-Derivat oder 1 Äquivalent A-Derivat und 2 Äquivalenten B-Derivat umsetzen. Man kann Cyanursäurechlorid aber auch mit je 1 Äquivalent eines A-Derivates und eines B-Derivates umsetzen und setzt das verbleibende Chlor mit einem Amin, Alkohol oder Phenol um.

Die für diese Umsetzung benötigten funktionellen Derivate vom A-Typ und B-Typ sind bekannte Verbindungen.

So sind z. B. Carboxylderivate von AI im US-Patent 3 766 205 beschrieben, Aminoderivate von AI im US-Patent 3 159 646 beschrieben. Carboxyl- oder Aminoderivate von AII sind in den US-Patenten 2 983 708, 3 208 865 oder 3 380 961 beschrieben.

Hydroxylderivate von Polyalkylpiperidinen sind beispielsweise in den DE-OS 2 352 658, 2 353 538 und 2 402 636 beschrieben, entsprechende Aminoderivate beispielsweise in den DE-OS 2 040 975 und 2 352 379. Carboxylderivate vom B-Typ sind beispielsweise in den DE-OS 2 337 865 und 2 719 133 beschrieben.

Verbindungen, in denen die Gruppe A mit Gruppe B durch eine direkte Bindung verknüpft ist, werden durch spezielle Methoden hergestellt. So erhält man beispielsweise durch Kupplung von 4-(4'-Hydroxyphenyl)-2,2,6,6-tetramethylpiperidin mit einer o-Nitrophenyldiazoniumverbindung die entsprechende o-Nitroazoverbindung, welche nach bekannten Reduktionsmethoden in das entsprechende Benztriazol übergeführt werden kann. Die hierfür benötigten Hydroxyphenylpiperidine sind in der DE-OS 2 258 086 beschrieben.

Weitere Einzelheiten zur Herstellung der erfindungsgemäßen Verbindungen sind in den später folgenden Beispielen angegeben.

Die neuen Verbindungen sind hervorragende Lichtschutzmittel für organische Materialien, die gegen Lichteinwirkung empfindlich sind, insbesondere für organische Polymere. Vor allem zeichnen sich die Verbindungen durch hohe Schutzwirkung gegen kurzwelliges Licht (UV-Licht) aus.

Polymere, die durch Zusatz der erfindungsgemäßen Verbindungen stabilisiert werden können, sind z. B. die folgenden:

1. Polymere von Mono- und Diolefinen, beispielsweise Polyäthylen (das gegebenenfalls vernetzt sein kann), Polypropylen, Polyisobutylen, Polymethylbuten-1, Polymethylpenten-1, Polyisopren oder Polybutadien.
2. Mischungen der unter 1) genannten Polymeren, z. B. Mischungen von Polypropylen mit Polyäthylen oder mit Polyisobutylen. Oder z. B. Mischungen von zwei Polyäthylenen von unterschiedlicher Dichte oder von unterschiedlichem Schmelzindex.
3. Copolymere von Mono- und Diolefinen, wie z. B. Äthylen-Propylen-Copolymere, Propylen-Buten-1-Copolymere, Propyen-Isobutylen-Copolymere, Äthylen-Buten-1-Copolymere sowie Terpolymere von Äthylen mit Propylen und einem Dien, wie Hexadien, Dicyclopentadien oder Äthylidennorbornen.
4. Polystyrol.
5. Copolymere von Styrol oder $\alpha$-Methylstyrol mit Dienen oder Acrylderivaten, wie z. B. Styrol-Butadien, Styrol-Acrylnitril, Styrol-Acrylnitril-Methylacrylat; Mischungen von hoher Schlagfähigkeit aus Styrol-Copolymeren und einem anderen Polymer wie z. B. einem Polyacrylat, einem Dien-Polymeren oder einem Äthylen-Propylen-Dien-Terpolymeren; sowie Block-Copolymere des Styrols, wie z. B. Styrol-Butadien-Styrol, Styrol-Isopren-Styrol oder Styrol-Äthylen/Butylen-Styrol.
6. Graft- oder Pfropfcopolymere von Styrol, wie z. B. Styrol auf Polybutadien, Styrol und Acrylnitril auf Polybutadien sowie deren Mischungen mit den unter 5) genannten Copolymeren, wie sie als sogenannte ABS-Polymere bekannt sind.
7. Halogenhaltige Polymere, wie z. B. Polyvinylchlorid, Polyvinylidenchlorid, Polyvinylfluorid, Polychloropren, Chlorkautschuke und Copolymere wie Vinylchlorid-Vinylidenchlorid, Vinylchlorid-Vinylacetat oder Vinylidenchlorid-Vinylacetat.
8. Polymere, die sich von $\alpha,\beta$-ungesättigten Säuren und deren Derivaten ableiten, wie Polyacrylate und Polymethacrylate, Polyacrylamide und Polyacrylnitril.
9. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyrat, Polyallylphthalat, Polyallylmelamin und deren Copolymere mit anderen Vinylverbindungen, wie Äthylen/Vinylacetat-Copolymere.

10. Homo- und Copolymere von Epoxyden, wie Polyäthylenoxyd, Polypropylenoxyd oder deren Copolymere mit Bisglycidyläthern.
11. Polyacetale, wie Polyoxymethylen, sowie solche Polyoxymethylene, die als Comonomeres Äthylenoxyd enthalten.
12. Polyphenylenoxyde.
13. Polyurethane und Polyharnstoffe.
14. Polycarbonate.
15. Polysulfone.
16. Polyamide und Copolyamide, die sich von Diaminen und Dicarbonsäuren und/oder von Aminocarbonsäuren oder den entsprechenden Lactamen ableiten, wie Polyamid 6, Polyamid 6/6, Polyamid 6/10, Polyamid 11, Polyamid 12.
17. Polyester, die sich von Dicarbonsäuren und Dialkoholen und/oder von Hydroxycarbonsäuren oder den entsprechenden Lactonen ableiten, wie Polyäthylenterephthalat, Polybutylenterephthalat, Poly-1,4-dimethylol-cyclohexanterephthalat und Copolyäther-ester.
18. Vernetzte Polymerisate, die sich von Aldehyden einerseits und Phenolen, Harnstoffen und Melaminen andererseits ableiten, wie Phenol-Formaldehyd-, Harnstoff-Formaldehyd- und Melamin-Formaldehydharze.
19. Alkydharze, wie Glycerin-Phthalsäure-Harze und deren Gemische mit Melamin-Formaldehydharzen.
20. Ungesättigte Polyesterharze, die sich von Copolyestern gesättigter und ungesättigter Dicarbonsäuren mit mehrwertigen Alkoholen sowie Vinylverbindungen als Vernetzungsmitteln ableiten, wie auch deren halogenhaltige, schwerbrennbare Modifikationen.
21. Vernetzte Epoxidharze, die sich von Polyepoxiden ableiten, z. B. von Bis-glycidyläthern oder von cycloaliphatischen Diepoxiden.
22. Natürliche Polymere, wie Cellulose, Gummi, Proteine sowie deren polymerhomolog chemisch abgewandelte Derivate, wie Celluloseacetate, -propionate und -butyrate, bzw. die Celluloseäther, wie Methylcellulose.

Von diesen Polymeren sind diejenigen von besonderem Interesse, die als Bindemittel für Lacke, insbesondere für Einbrennlacke verwendet werden, wie z. B. diejenigen der Gruppen 18, 19, 20 und 21 der obigen Aufzählung. Es hat sich gezeigt, daß in Lacken eine Kombination von Piperidin-Lichtschutzmitteln mit UV-Absorbern sehr wertvoll ist. Bei Einbrennlacken stellt hierbei die Flüchtigkeit der üblichen niedermolekularen Lichtschutzmittel auf Basis von Benztriazolen, Benzophenonen und Polyalkylpiperidinen ein ernstliches technisches Problem dar. Durch die chemische Kombination der verschiedenen Typen von Lichtschutzmitteln gemäß der vorliegenden Erfindung wird die Flüchtigkeit stark herabgesetzt, so daß die neuen Verbindungen sich auch für die Stabilisierung von Einbrennlacken eignen.

Die erfindungsgemäßen Verbindungen werden den Polymeren in einer Menge von 0,05 bis 5 Gew.-%, vorzugsweise 0,1 bis 1 Gew.-%, zugesetzt.

Der Zusatz der erfindungsgemäßen Lichtschutzmittel zum Polymeren erfolgt vor dessen Formgebung durch Vermischen mit dem pulverförmigen Polymeren oder mit der Schmelze oder Lösung des Polymeren oder seiner Vorprodukte. Gleichzeitig können dem Polymeren auch andere Zusätze einverleibt werden, wie sie in der Kunststofftechnologie bekannt und üblich sind. Dies können z. B. Antioxydantien, Metalldesaktivatoren sonstige Stabilisatoren, Weichmacher, Gleitmittel, Treibmittel, Pigmente, Füllstoffe oder sonstige Hilfsmittel sein. Einzelne Beispiele solcher bekannter und üblicher Kunststoffzusätze sind in der DE-OS 2 349 962, Seiten 25—32, aufgeführt.

Die so stabilisierten Polymeren können in verschiedenster Form verwendet werden, z. B. als Folien, Fasern, Bändchen, Profile oder als Bindemittel für Lacke, Klebemittel oder Kitte.

Die Herstellung und Verwendung der erfindungsgemäßen Verbindungen wird in den folgenden Beispielen beschrieben. Teile bedeuten darin Gewichtsteile und % Gewichtsprozente. Die Temperaturen sind in °C angegeben.

## Beispiel 1

46,7 g 2-(2-Hydroxy-3-methyl-5-$\beta$-methoxycarbonyläthyl-phenyl)-benztriazol (hergestellt nach US-Patent 3 766 205) werden mit 23,6 g 2,2,6,6-Tetramethyl-4-hydroxypiperidin und 9 g Natriummethylat in 300 ml Xylol 20 Stunden unter schwachem Stickstoffstrom auf 130—135° erhitzt. Dabei wird das entstehende Methanol fortlaufend abdestilliert. Das Reaktionsgemisch wird auf Raumtemperatur abgekühlt, mit Essigsäure angesäuert und dreimal mit je 100 ml Wasser extrahiert. Die vereinigten wäßrigen Extrakte werden mit Ammoniak alkalisch gestellt, der entstandene Niederschlag abfiltriert, mit Wasser gewaschen, getrocknet und aus Ligroin kristallisiert. Man erhält das 2-{2-Hydroxy-3-methyl-5-[$\beta$-(2,2,6,6-tetramethyl-4-piperidinyloxycarbonyl)-äthyl]-phenyl}-benztriazol (Verbindung 1) als fast farbloses Pulver vom Smp. 131°.

Verwendet man anstelle von 2,2,6,6-Tetramethyl-4-hydroxypiperidin eine äquivalente Menge 1,2,2,6,6-Pentamethyl-4-hydroxypiperidin bzw. 1-Benzyl-2,2,6,6-tetramethyl-4-hydroxypiperidin und verfährt man im übrigen wie oben beschrieben, so erhält man das 2-{2-Hydroxy-3-methyl-5-[β-(1,2,2,6,6-pentamethyl-4-piperidinyloxycarbonyl)äthyl]-phenyl}-benztriazol (Verbindung 2) vom Smp. 110–111° bzw. das 2-{2-Hydroxy-3-methyl-5-[β-(1-benzyl-2,2,6,6-tetramethyl-4-piperidinyloxycarbonyl)-äthyl]-phenyl}-benztriazol (Verbindung 3) vom Smp. 127–128°.

## Beispiel 2

23,1 g 2-(2-Hydroxy-3,5-di-tert.-butyl-phenyl)-benztriazol-5-carbonsäurechlorid werden bei Raumtemperatur in 300 ml Toluol vorgelegt. Zu dieser Lösung tropft man in ca. 30 Minuten eine Lösung von 10,1 g 2,2,6,6-Tetramethyl-4-aminopiperidin in 100 ml Toluol und rührt anschließend das Reaktionsgemisch während 15 Stunden bei Raumtemperatur. Das ausgefallene Hydrochlorid wird abfiltriert und in 200 ml Äthanol und 200 ml Wasser gelöst. Die Lösung wird mit Natriumcarbonat alkalisch gestellt, der entstandene Niederschlag abfiltriert, mit Wasser gewaschen, getrocknet und aus Toluol kristallisiert. Man erhält das 2-(2-Hydroxy-3,5-di-tert.-butyl-phenyl)-benztriazol-5-carbonsäure-N-(2,2,6,6-tetramethyl-4-piperidinyl)-amid (Verbindung 4) vom Smp. 267°.

Verwendet man anstelle von 2,2,6,6-Tetramethyl-4-aminopiperidin eine äquivalente Menge 2,2,6,6-Tetramethyl-4-β-aminoäthyl-piperidin und verfährt man in übrigen wie oben beschrieben, so erhält man das 2-(2-Hydroxy-3,5-di-tert-butyl-phenyl)-benztriazol-5-carbonsäure-N-[β-(2,2,6,6-tetramethyl-4-piperidinyl)-äthyl]-amid (Verbindung 5) vom Smp. 228°.

## Beispiel 3

57,2 g 2-Hydroxy-4-methoxycarbonyl-methyloxy-benzophenon werden mit 36 g 1,2,2,6,6-Pentamethyl-4-hydroxypiperidin und 15 g Natriummethylat 8 Stunden in 250 ml Xylol unter schwachem Stickstoffstrom auf 130–135° erhitzt. Das entstehende Methanol wird während der Umesterung fortlaufend abdestilliert. Nach dem Abkühlen auf Raumtemperatur wird das Reaktionsgemisch mit Essigsäure angesäuert und dann dreimal mit je 100 ml Wasser extrahiert. Die vereinigten wäßrigen Extrakte werden mit Ammoniak alkalisch gestellt, der Niederschlag abfiltriert, mit Wasser gewaschen, getrocknet und aus Äthylenglykolmonomethyläther umkristallisiert. Man erhält das 2-Hydroxy-4-(1,2,2,6,6-pentamethyl-piperidinyloxy-carbonylmethoxy-benzophenon (Verbindung 6) vom Smp. 79°.

## Beispiel 4

12,5 g o-Nitranilin werden nach bekannten Verfahren diazotiert und die erhaltene Diazoniumsalzlösung zu einer Lösung von 16,3 g 2,2,6,6-Tetramethyl-4-(4′-hydroxyphenyl)-piperidin in 100 ml Wasser und 4 g Natriumhydroxyd bei 0–5° und einem pH von 10–12 getropft. Nach beendeter Kupplung stellt man das Reaktionsgemisch mit Essigsäure auf pH 7 und filtriert den Azofarbstoff ab. Nach dem Trocknen bei 50° im Vakuum wird der Azofarbstoff (19,1 g) in 50 ml Äthylenglykolmonomethyläther gelöst. Dazu gibt man eine Lösung von 6 g Natriumhydroxyd in 20 ml Wasser und erwärmt auf 80°. Bei dieser Temperatur tropft man in ca. 15 Minuten 2,5 g Hydrazinhydrat zu und rührt anschließend 1 Stunde bei 80° nach. Dabei tritt eine Farbänderung des Reaktionsgemisches von violett nach braun ein. Man kühlt auf Raumtemperatur ab und gibt portionsweise 6,5 g Zinkstaub in ca. 30 Minuten hinzu. Danach wird das Reaktionsgemisch auf 60° erwärmt, nochmals mit 6,5 g Zinkstaub und 25 ml 10 n Natronlauge versetzt und 1 Stunde nachgerührt. Das hellgrüne Reaktionsgemisch wird vom Zinkschlamm filtriert und das Filtrat langsam mit ca. 70 ml Eisessig versetzt (pH ~ 6), die erhaltene Lösung geklärt und dann mit Ammoniak alkalisch gestellt. Der erhaltene Niederschlag wird abfiltriert, mit Wasser gewaschen, getrocknet und aus Ligroin kristallisiert. Man erhält das 2-[2-Hydroxy-5-(2,2,6,6-tetramethylpiperidin-4-yl)-phenyl]-benztriazol (Verbindung 7) vom Smp. 178–180°.

Verwendet man anstelle von 2,2,6,6-Tetramethyl-4-(4′-hydroxyphenyl)-piperidin eine äquivalente Menge 2,2,6,6-Tetramethyl-4-(3′-methyl-4′-hydroxyphenyl)-piperidin bzw. 2,2,6,6-Tetramethyl-4-(3′-t-butyl-4′-hydroxyphenyl)-piperidin bzw. 2,2,6,6-Tetramethyl-4-(3′-cyclohexyl-(4′-hydroxyphenyl)-piperidin bzw. 2,2,6,6-Tetramethyl-4-(2′-hydroxy-5′-methylphenyl)-piperidin und verfährt man im übrigen wie oben beschrieben, so erhält man das 2-[2-Hydroxy-3-methyl-5-(2,2,6,6-tetramethylpiperidin-4-yl)-phenyl]-benztriazol (Verbindung 8) vom Smp. 219–220° bzw. das 2-[2-Hydroxy-3-t-butyl-5-(2,2,6,6-tetramethylpiperidin-4-yl)-phenyl]-benztriazol (Verbindung 9) vom Smp. 162–64° bzw. das 2-[2-Hydroxy-3-cyclohexyl-5-(2,2,6,6-tetramethylpiperidin-4-yl)-phenyl]-benztriazol (Verbindung 10) vom Smp. 155–156° bzw. das 2-[2-Hydroxy-3-(2,2,6,6-tetramethylpiperidin-4-yl)-5-methyl-phenyl]-benztriazol (Verbindung 11) vom Smp. 168–69°.

Beispiel 5

20 g 2-[2-Hydroxy-3-methyl-5-(2,2,6,6-tetramethylpiperidin-4-yl)-phenyl]-benztriazol (Verbindung 8) werden in 150 ml Ameisensäure (98%ig) gelöst. Darauf läßt man innerhalb von ca. 1 Stunde, bei Raumtemperatur, 87,4 ml einer 35%igen Formaldehydlösung zutropfen. Die leicht bräunliche Lösung wird anschließend 20 Stunden am Rückfluß erhitzt, auf Raumtemperatur abgekühlt und auf eine kalte, gesättigte Kaliumcarbonatlösung getropft. Der weiße, kristalline Niederschlag wird abfiltriert, mit Wasser gewaschen, getrocknet und aus Ligroin umkristallisiert. Man erhält das 2-[2-Hydroxy-3-methyl-5-(1,2,2,6,6-pentamethylpiperidin-4-yl)-phenyl]-benztriazol vom Smp. 206–207° (Verbindung 12).

Beispiel 6

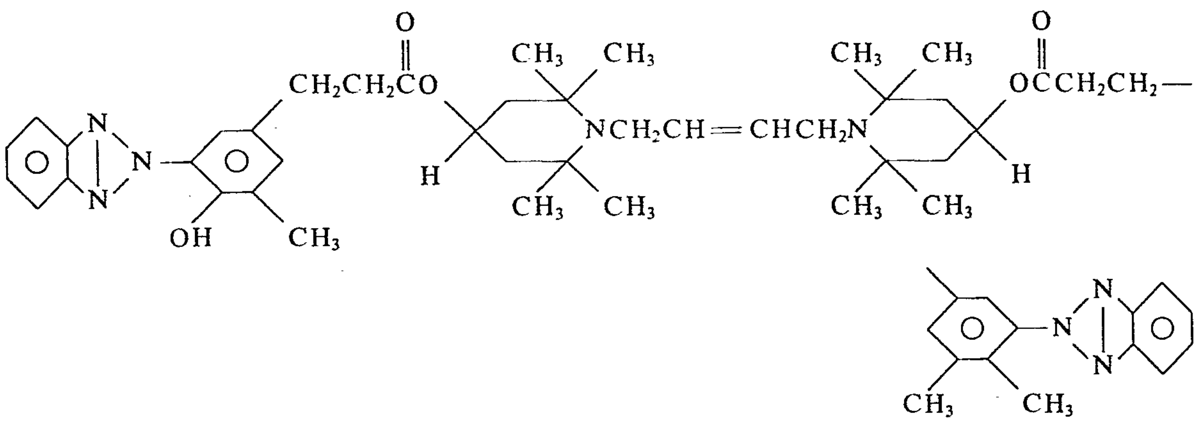

130,7 g 2-(2-Hydroxy-3-methyl-5-β-methoxycarbonyl)-äthyl-phenyl)-benztriazol und 73,3 g 1,4-Bis-(2,2,6,6-tetramethyl-4-hydroxypiperidinyl-1)-2-buten werden in 600 ml Xylol nach Zugabe von 1,0 g Tetrabutylorthotitanat 24 Stunden unter schwachem Stickstoffstrom auf 130–35° erhitzt. Dabei wird das entstehende Methanol fortlaufend abdestilliert. nach dieser Zeit wird 1,0 g Tetrabutylorthotitanat zugegeben und weitere 24 Stunden bei 130–35° umgeestert. Nun wird das Xylol weitgehend abdestilliert und der Rückstand zweimal aus Ligroin umkristallisiert. Man erhält den Diester von obiger Konstitution (Verbindung 13) als fast farblose Kristalle vom Smp. 135°.

Beispiel 7

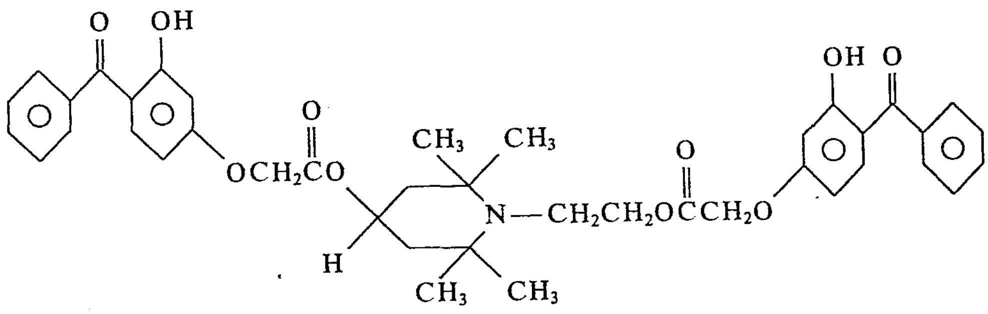

30,0 g 2-Hydroxy-4-methoxycarbonyl-methyloxybenzophenon werden mit 10,0 g 1-Hydroxyäthyl-2,2,6,6-tetramethyl-4-hydroxypiperidin und 0,5 g Tetrabutylorthotitanat 15 Stunden in 200 ml Xylol unter schwachen Stickstoffstrom auf 130–35° erhitzt. Das entstehende Methanol wird während der Umesterung laufend abdestilliert. Nach der Zugabe einer neuen Portion von 0,5 g Tetrabutylorthotitanat wird weitere 15 Stunden umgeestert. Die Reaktionslösung wird geklärt und eingedampft und der Rückstand aus Toluol/Hexan umkristallisiert. Man erhält den Diester mit der oben angegebenen Konstitution (Verbindung 14) als gelbliche Kristalle vom Smp. 130°.

Beispiel 8

Lichtstabilisierung eines 2-Schicht-Metallic-Lackes

1) Rezepturen

a) Basislack (pigmentiert)

| | |
|---|---|
| 54,4 g | Polyester L 1850 (Dynamit Nobel AG, Troisdorf, BRD) |
| 5,5 g | Melaminharz Maprenal® MF 650 (Cassella-Hoechst AG, Wiesbaden, BRD) |
| 3,7 g | Toluol |
| 2,8 g | n-Butanol |
| 3,7 g | Butylacetat |
| 20,7 g | Alcoa® 726 (Alcoa GmbH, Frankfurt/M, BRD) 40%ige Dispersion von Aluminium-Bronze in Xylol/Butylacetat 1 : 1 |
| 9,2 g | Celluloseacetobutyrat EAB 551-0.01 (Eastman Chem. Chem. Corp., Zug, Schweiz) |
| 100 g | |

Vor dem Auftrag wurde der Basislack mit einem Xylol/Butylacetat-Gemisch 1 : 1 auf eine Viskosität von 15 Sekunden Auslaufzeit im Ford-Becher, Typ 4, verdünnt.

b) Klarlack (Decklack)

| | |
|---|---|
| 100 g | Acrylharz auf Basis eines Hydroxyäthyl-acrylat-Copolymerisates |
| 30 g | Harz-Härter (Dosmodur®N, Bayer A. G., Leverkusen, (BRD) |
| 3 g | Verlaufhilfsmittel (Byketol®Spezial, Byk-Mallinckrodt, Wesel, BRD) |
| 53 g | Lösungsmittelgemisch Äthylglykolacetat/Butylacetat/Xylol 30 : 30 : 40 |
| 186 g | |

Der Härter wurde erst kurz vor dem Auftrag zugemischt. Hierbei wurden auch die im Abschnitt 5 genannten Stabilisatoren zugegeben.

2) Bereitung der Proben

Aluminiumbleche von 56 × 67 mm wurden mit einem Haftgrund (Füllprimer der Fa. Dr. Herberts) beschichtet und 15 Minuten bei 175° eingebrannt.

Hierauf wurde der pigmenthaltige Basislack in einer Schichtdicke von ca. 15 µm aufgesprüht und nach kurzer Trocknung bei Raumtemperatur wurde der Klarlack in einer Schichtdicke von ca. 35 µm aufgesprüht. Der 2-Schicht-Lack wurde bei 80° C 30 Minuten eingebrannt und die Probe 4 Wochen im Normklima (23° C/50% RLF) gelagert.

3) Bewitterung

a) Schnellbewitterung im QUV-Bewitterungsgerät der Fa. Q-Panel Corp., USA, gemäß ASTM G 53-77. Hierbei wurden die Proben abwechselnd 4 Stunden trocken bei 60° C und 4 Stunden feucht bei 50° C bewittert. Insgesamt wurde 900 Stunden bewittert.
b) Florida-Bewitterung. Die Proben wurden 12 Monate in einer Blackbox mit 5° Neigung gegen Süden im Freien bewittert.

4) Bewertung der Proben

Gemessen wurde
a) die Glanz-Reflexion unter einem Winkel von 20° gemäß DIN 67 530 bzw. ASTM D 523.
b) die Rißbildung gemäß TNO-Rißbildskala.
c) die Gesamt-Farbtondifferenz $\Delta E$ gemäß DIN 6174.
d) die Farbänderung mittels Grauskala gemäß ISO-Recom. R 105/Part 2.

5) Verwendete Lichtschutzmittel

LS I

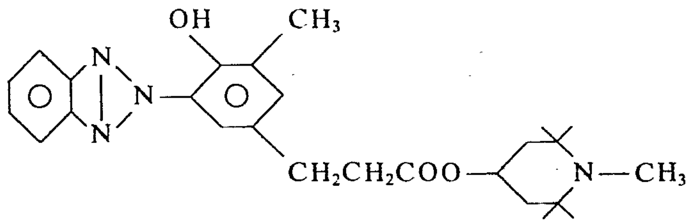

(Verbindung 2 des Beispiels 1)

LS II

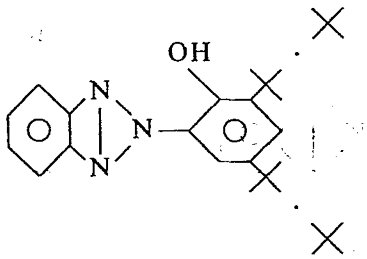

(Handelsüblicher UV-Absorber)

LS III

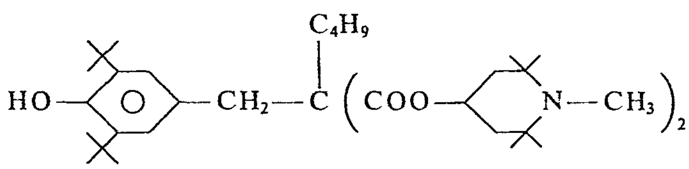

(Handelsübliches Lichtschutzmittel auf Polyalkylpiperidinbasis)

6) Resultate

a) Schnellbewitterung

| Lichtschutzmittel*) | Glanz-Reflexion vor Bewitterung | nach | Farbton-differenz $\Delta_E$ | Rißbild**) |
|---|---|---|---|---|
| Ohne | 95 | 84 | 9,4 | F 7c |
| 1% LS I | 96 | 96 | 4,5 | 0 |
| 2% LS I | 96 | 96 | 3,0 | 0 |
| 1% LS II + 1% LS III | 94 | 98 | 4,7 | F 4a |

b) Florida-Bewitterung

| Lichtschutzmittel*) | Glanz-Reflexion vor Bewitterung | nach | Farbänderung (Grauton) | Rißbild**) |
|---|---|---|---|---|
| Ohne | 95 | 21 | 3 | F 8 |
| 1% LS I | 96 | 83 | 4 | 0 |
| 2% LS I | 96 | 85 | 4 | 0 |
| 1% LS II + 1% LS III | 94 | 73 | 4 | 0 |

*) Die angegebene Menge Lichtschutzmittel ist auf den Feststoffgehalt (Harz + Härter) des Klarlackes berechnet.
**) Rißbild 0 bedeutet: keine sichtbare Rißbildung.

**Patentansprüche**

1. Verbindungen, die eine oder zwei Gruppen der Formel A I oder A II

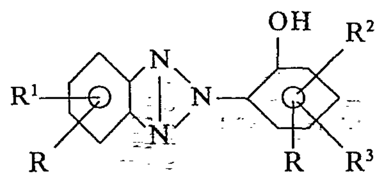

(A I)

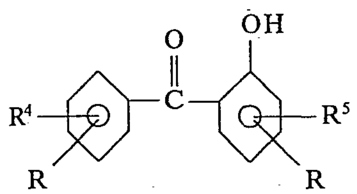

(A II)

und eine oder zwei der Gruppen B I bis B XIII enthalten,

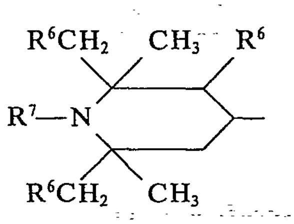

(B I)

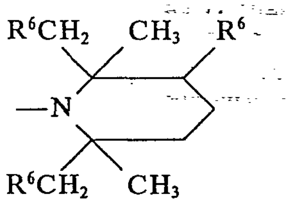

(B II)

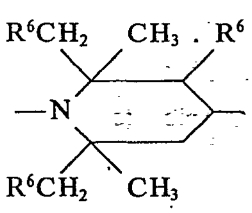

(B III)

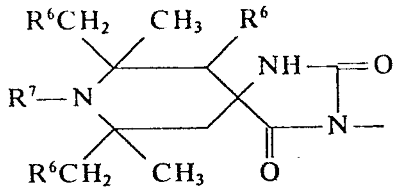

(BIV)

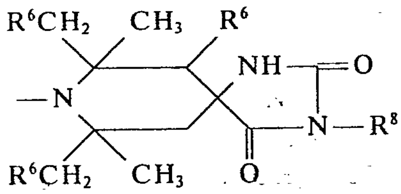

(BV)

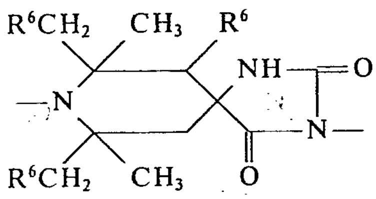

(BVI)

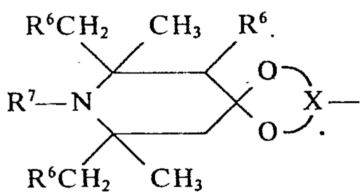

(BVII)

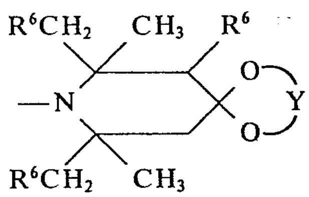

(BVIII)

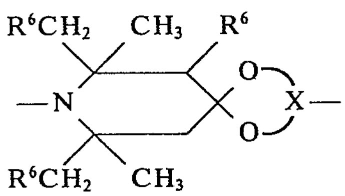

(BIX)

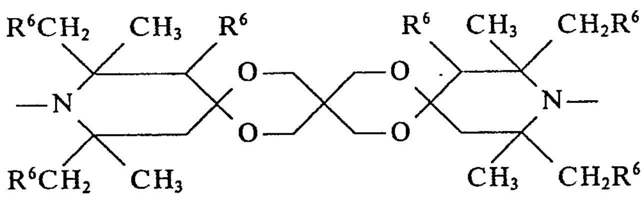

(BX)

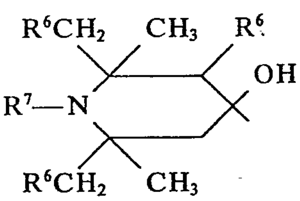

(BXI)

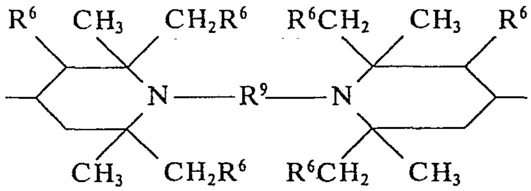

(BXII)

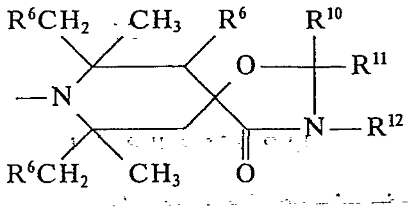

(BXIII)

worin R Wasserstoff oder eine freie Valenz,

$R^1$ Wasserstoff, Chlor, $C_1$–$C_4$-Alkyl, OH oder $C_1$–$C_4$-Alkoxy,

$R^2$ und $R^3$ unabhängig voneinander H, Cl, $C_1$–$C_{12}$-Alkyl, $C_5$–$C_8$-Cycloalkyl, Phenyl, $C_7$–$C_9$-Phenylalkyl, OH oder $C_1$–$C_{12}$-Alkoxy,

$R^4$ und $R^5$ unabhängig voneinander H, Cl, OH, $C_1$–$C_{12}$-Alkoxy oder $C_1$–$C_{12}$-Alkyl,

$R^6$ H oder $CH_3$,

$R^7$ H, O˙, $C_1$–$C_{12}$-Alkyl, $C_3$–$C_8$-Alkenyl, Benzyl, Acetyl oder eine Gruppe

$$—CH_2—CH(OH)—R^{13}$$

$R^8$ $C_1$–$C_{12}$-Alkyl, $C_3$–$C_8$-Alkenyl oder $C_7$–$C_9$-Phenylalkyl,

$R^9$ eine Gruppe

$$—CH_2CH{=}CHCH_2—$$

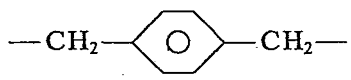

oder

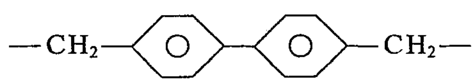

$R^{10}$ und $R^{11}$ Wasserstoff, $C_1$–$C_{12}$-Alkyl, Phenyl oder Benzyl, oder

$R^{10}$ und $R^{11}$ zusammen $C_4$–$C_{19}$-Alkylen,

$R^{12}$ H oder eine freie Valenz,

$R^{13}$ H, $CH_3$, $C_2H_5$ oder Phenyl,

X eine Gruppe

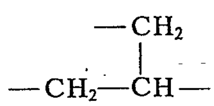

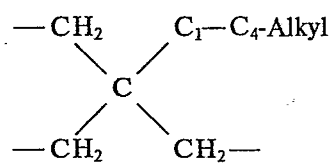

und

Y eine $C_2$–$C_8$-Alkylengruppe bedeuten,

wobei die Gruppen A mit den Gruppen B durch eine direkte Bindung oder durch ein zweiwertiges Zwischenglied –Z– verbunden sind und –Z– eine der folgenden Gruppen sein kann:

$$—(CH_2)_m—CO—O— \quad —(CH_2)_m—CO—O—R^{14}— \quad —O—(CH_2)_n—CO—O—R^{14}—$$

$$—CH_2—CH(OH)—CH_2—O—CO—(CH_2)_m—$$

$$—O—CH_2—CH(OH)—CH_2—O—CO—(CH_2)_m—$$

$$—CH_2—CH(OH)—CH_2— \quad —CH_2—CH(OH)—CH_2—O—$$

$$—CH_2—CH(OH)—CH_2—N(R^{15})—(CH_2)_m—$$

$$—O—CH_2—CH(OH)—CH_2—N(R^{15})—(CH_2)_m—$$

$$—N(R^{15})—CH_2—CH(OH)—CH_2—N(R^{16})—(CH_2)_m—$$

$-CH_2-CH(R^{13})-O-CH_2-CH(OH)-CH_2-N(R^{15})-(CH_2)_m-$

$-CH_2-CH(R^{13})-O-CH_2-CH(OH)-CH_2-O-CO-(CH_2)_m-$

$-(CH_2)_m-N(R^{15})-CO-$ $\quad$ $-(CH_2)_m-N(R^{15})-CO-(CH_2)_n-$

$-O-(CH_2)_3-N(R^{15})-CO-(CH_2)_m-$ $\quad$ $-CH_2-NH-CO-CH_2-O-$

$-CH_2-NH-CO-CH_2-O-CH(R^{13})-CH_2-$

$-CH_2-NH-CO-CH_2-N(R^{15})-(CH_2)_m-$

$-CH_2-NH-CO-CH_2CH_2-N(R^{15})-(CH_2)_m-$

oder

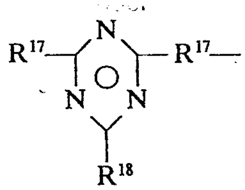

worin m 0, 1 oder 2 ist, n 1 oder 2 ist, $R^{14}$ $-CH_2-$ oder $-CH(R^{13})-CH_2-$ bedeutet, $R^{15}$ und $R^{16}$ H, $C_1-C_{12}$-Alkyl, Cyclohexyl oder Benzyl bedeuten, $R^{17}$ $-O-$ oder $-NR^{15}-$ darstellt und $R^{18}$ $C_1-C_{12}$-Alkoxy, Phenoxy oder eine Aminogruppe

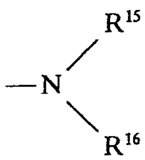

bedeutet, oder die Gruppen A und B durch einen dreiwertigen Rest der Formel

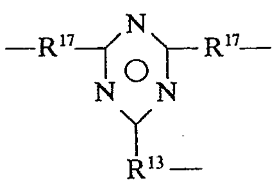

verbunden sind

oder eine Gruppe AII mit einer Gruppe B über einen Rest $-O-(CH_2)_n-CO-O-$ verbunden ist.

2. Verbindungen gemäß Anspruch 1, die eine oder zwei der Gruppen B I bis B VI enthalten.

3. Verbindungen gemäß Anspruch 2, worin $R^6$ Wasserstoff, $R^7$ Wasserstoff, $C_1-C_8$ Alkyl, Allyl oder Benzyl und $R^8$ $C_1-C_{12}$ Alkyl, $C_3-C_5$ Alkenyl oder Benzyl ist.

4. Verbindungen gemäß Anspruch 1, die eine Gruppe der Formel A I enthalten, worin $R^1$ Wasserstoff oder Chlor ist.

5. Verbindungen gemäß Anspruch 1, in denen die Gruppen A mit den Gruppen B durch eine direkte Bindung oder eine der Gruppen $-(CH_2)_m-CO-O-$, $-(CH_2)_m-CO-O-R^{14}-$, $-O-(CH_2)_n-CO-O-R^{14}-$, $-(CH_2)_m-N(R^{15})-CO-$, $-(CH_2)_m-N(R^{15})-CO-(CH_2)_n-$, $-CH_2-NH-CO-CH_2-O-$ oder $-CH_2-NH-CO-CH_2-N(R^{15})-(CH_2)_m-$ verbunden sind.

6. Verbindungen gemäß Anspruch 1, in denen die Gruppen A mit den Gruppen B durch einen Rest

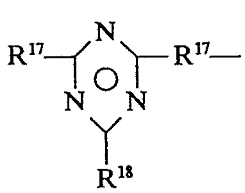 oder 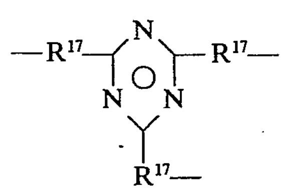

verbunden sind, worin $R^{17}$ eine Gruppe $NR^{15}$ darstellt und $R^{15}$ die in Anspruch 1 gegebene Bedeutung hat.

7. Verwendung von Verbindungen gemäß Anspruch 1 als Stabilisatoren für organische Polymere gegen Schädigung durch Lichteinwirkung.

8. Verwendung gemäß Anspruch 7, dadurch gekennzeichnet, daß das organische Polymer ein Bindemittel für Lacke ist.

9. Gegen Lichtschädigung stabilisiertes organisches Polymer, dadurch gekennzeichnet, daß es als Stabilisator 0,05 bis 5 Gew.-% einer Verbindung des Anspruchs 1 enthält.

10. Stabilisiertes Polymer gemäß Anspruch 9, dadurch gekennzeichnet, daß das Polymer ein Bindemittel für Lacke ist.

11. Verbindungen gemäß Anspruch 1, in denen eine Gruppe AII mit einer Gruppe B über einen Rest $-O-(CH_2)_n-CO-O-$ verbunden ist.

**Claims**

1. A compound which contains one or two groups of the formula A I or A II

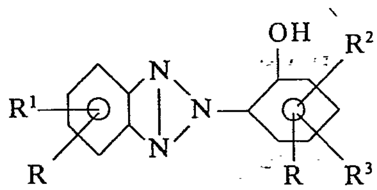

(A I)

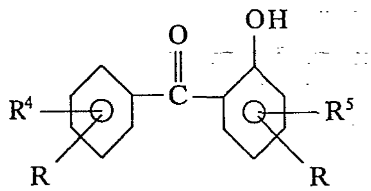

(A II)

and one or two of the groups B I to B XIII

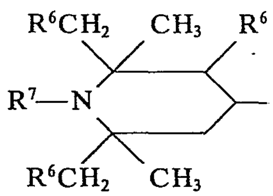

(BI)

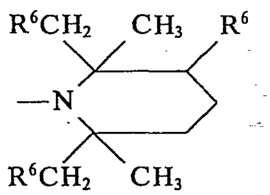

(BII)

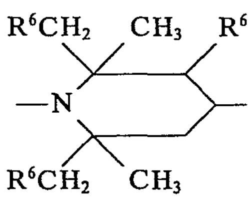

(BIII)

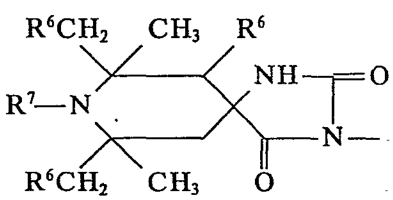

(BIV)

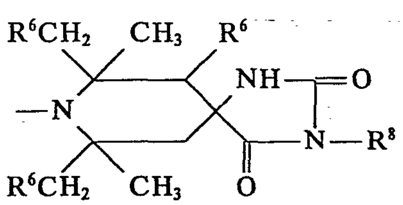

(BV)

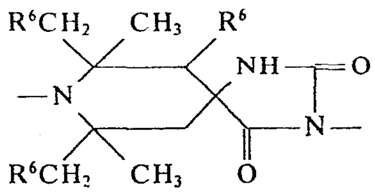

(B VI)

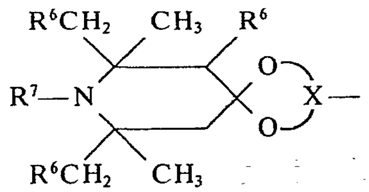

(B VII)

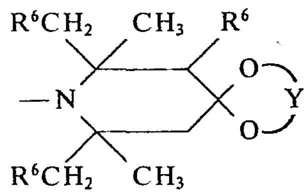

(B VIII)

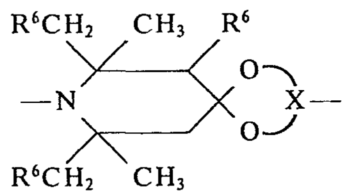

(B IX)

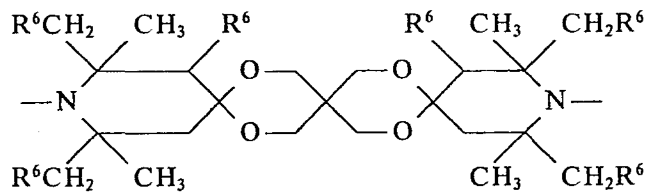

(B X)

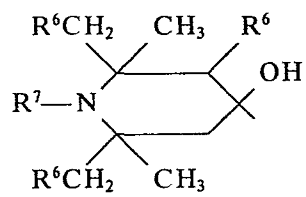

(B XI)

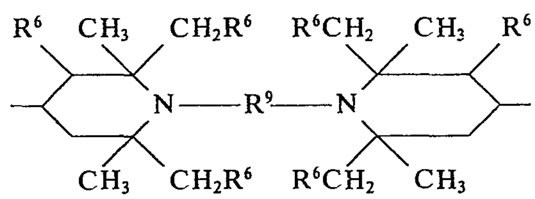

(B XII)

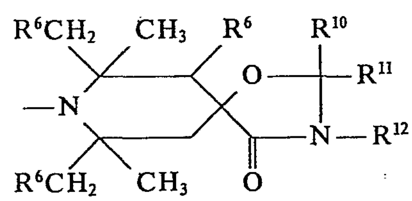

(B XIII)

in which R is hydrogen or a free valency, $R^1$ is hydrogen, chlorine, $C_1$–$C_4$ alkyl, OH or $C_1$–$C_4$ alkoxy, $R^2$ and $R^3$ independently of one another are H, Cl, $C_1$–$C_{12}$ alkyl, $C_5$–$C_8$ cycloalkyl, phenyl, $C_7$–$C_9$ phenylalkyl, OH or $C_1$–$C_{12}$ alkoxy, $R^4$ and $R^5$ independently of one another are H, Cl, OH, $C_1$–$C_{12}$ alkoxy or $C_1$–$C_{12}$ alkyl, $R^6$ is H or $CH_3$, $R^7$ is H, O$^\cdot$, $C_1$–$C_{12}$ alkyl, $C_3$–$C_8$ alkenyl, benzyl, acetyl or a group

$-CH_2-CH(OH)-R^{13}$

$R^8$ is $C_1$–$C_{12}$ alkyl, $C_3$–$C_8$ alkenyl or $C_7$–$C_9$ phenylalkyl, $R^9$ is a

$CH_2CH=CHCH_2-$

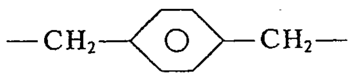 or 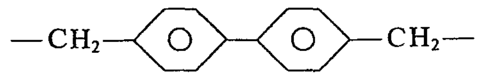

group, $R^{10}$ and $R^{11}$ are hydrogen, $C_1$–$C_{12}$ alkyl, phenyl or benzyl, or $R^{10}$ and $R^{11}$ are together $C_4$–$C_{19}$ alkylene, $R^{12}$ is H or a free valency, $R^{13}$ is H, $CH_3$, $C_2H_5$ or phenyl, X is a

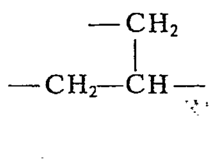 or 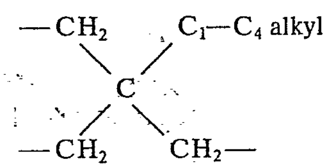

group and Y is a $C_2$–$C_8$ alkylene group, the groups A being linked to the groups B by a direct bond or by a divalent linking member –Z– and –Z– can be one of the following groups:

$-(CH_2)_m-CO-O-$ $-(CH_2)_m-CO-O-R^{14}-$ $-O-(CH_2)_n-CO-O-R^{14}-$

$-CH_2-CH(OH)-CH_2-O-CO-(CH_2)_m-$

$-O-CH_2-CH(OH)-CH_2-O-CO-(CH_2)_m-$

$-CH_2-CH(OH)-CH_2-$ $-CH_2-CH(OH)-CH_2-O-$

$-CH_2-CH(OH)-CH_2-N(R^{15})-(CH_2)_m-$

$-O-CH_2-CH(OH)-CH_2-N(R^{15})-(CH_2)_m-$

$-N(R^{15})-CH_2-CH(OH)-CH_2-N(R^{16})-(CH_2)_m-$

$-CH_2-CH(R^{13})-O-CH_2-CH(OH)-CH_2-N(R^{15})-(CH_2)_m-$

$-CH_2-CH(R^{13})-O-CH_2-CH(OH)-CH_2-O-CO-(CH_2)_m-$

$-(CH_2)_m-N(R^{15})-CO-$ $-(CH_2)_m-N(R^{15})-CO-(CH_2)_n-$

$-O-(CH_2)_3-N(R^{15})-CO-(CH_2)_m-$ $-CH_2-NH-CO-CH_2-O-$

$-CH_2-NH-CO-CH_2-O-CH(R^{13})-CH_2-$

$-CH_2-NH-CO-CH_2-N(R^{15})-(CH_2)_m-$

$-CH_2-NH-CO-CH_2CH_2-N(R^{15})-(CH_2)_m-$

or

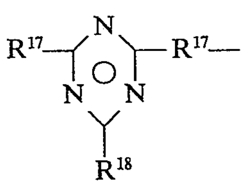

in which m is 0, 1 or 2, n is 1 or 2, $R^{14}$ is $-CH_2-$ or $-CH(R^{13})-CH_2-$, $R^{15}$ and $R^{16}$ are H, $C_1$–$C_{12}$ alkyl, cyclohexyl or benzyl, $R^{17}$ is –O– or $-NR^{15}-$ and $R^{18}$ is $C_1$–$C_{12}$ alkoxy, phenoxy or an amino group

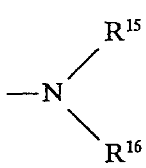

or the groups A and B are linked by a trivalent radical of the formula

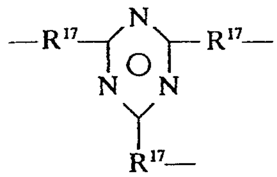

or a group AII is linked to a group B via a radical $-O-(CH_2)_n-CO-O-$.

2. A compound according to claim 1, which contains one or two of the groups B I to B VI.

3. A compound according to claim 2, in which $R^6$ is hydrogen, $R^7$ is hydrogen, $C_1-C_8$ alkyl, allyl or benzyl and $R^8$ is $C_1-C_{12}$ alkyl, $C_3-C_5$ alkenyl or benzyl.

4. A compound according to claim 1, which contains a group of the formula A I, in which $R^1$ is hydrogen or chlorine.

5. A compound according to claim 1, in which the groups A are linked to the groups B by a direct bond or one of the groups $-(CH_2)_m-CO-O-$, $-(CH_2)_m-CO-O-R^{14}-$, $-O-(CH_2)_n-CO-O-R^{14}-$, $-(CH_2)_m-N(R^{15})-CO-$, $-(CH_2)_m-N(R^{15})-CO-(CH_2)_n-$, $-CH_2-NH-CO-CH_2-O-$ or $-CH_2-NH-CO-CH_2-N(R^{15})-(CH_2)_m-$.

6. A compound according to claim 1, in which the groups A are linked to the groups B by a radical

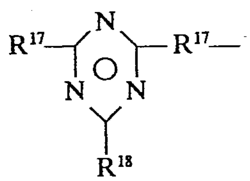 or 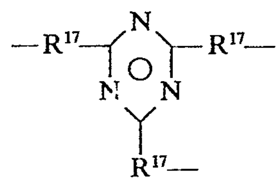

in which $R^{17}$ is a group $NR^{15}$ and $R^{15}$ is as defined in claim 1.

7. The use of a compound according to claim 1 as a stabiliser for organic polymers against damage caused by the action of light.

8. Use according to claim 7, wherein the organic polymer is a binder for lacquers.

9. An organic polymer stabilised against damage caused by light, which contains 0.05 to 5% by weight of a compound of claim 1 as a stabiliser.

10. A stabilised polymer according to claim 9, in which the polymer is a binder for lacquers.

11. A compound according to claim 1, in which a group AII is linked to a group B through a radical $-O-(CH_2)_n-CO-O-$.

## Revendications

1. Composés contenant un ou deux des radicaux répondant aux formules A I et A II

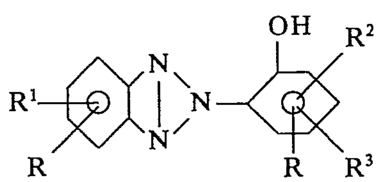

(A I)

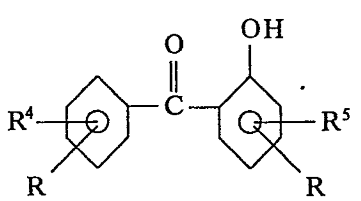

(A II)

et un ou deux des radiaux répondant aux formules B I à B XIII:

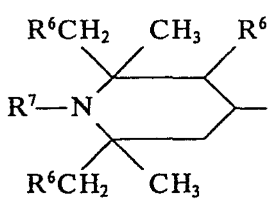

(BI)

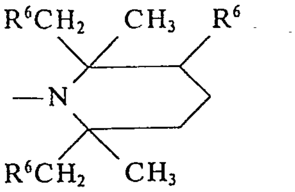

(BII)

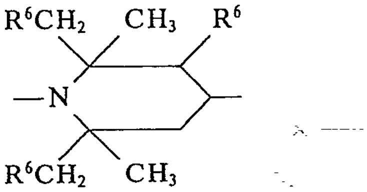

(BIII)

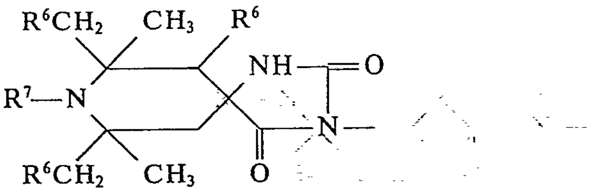

(BIV)

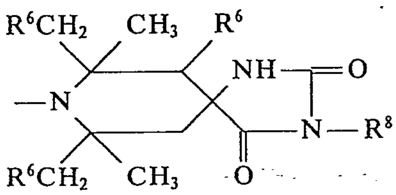

(BV)

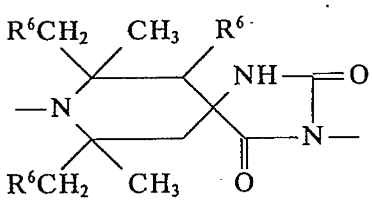

(BVI)

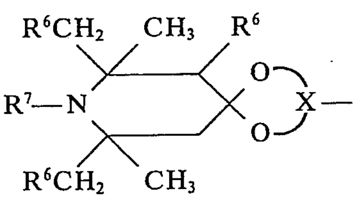

(BVII)

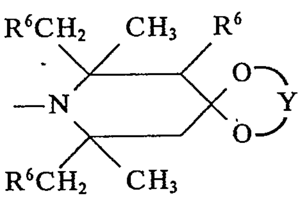

(BVIII)

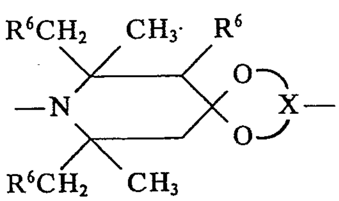

(BIX)

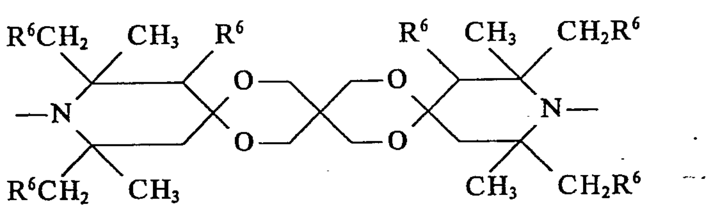

(BX)

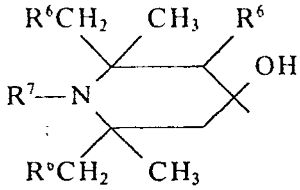

(BXI)

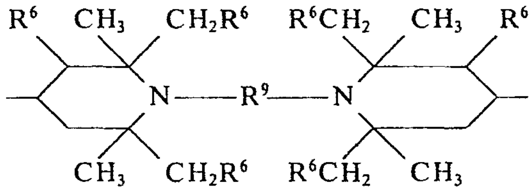

(BXII)

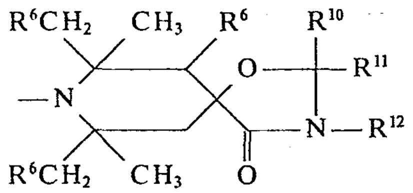

(BXIII)

dans lesquelles

R représente l'hydrogène ou une valence libre,

$R^1$ représente l'hydrogène, le chlore, un alkyle en $C_1$–$C_4$, –OH ou un alcoxy en $C_1$–$C_4$,

$R^2$ et $R^3$ représentent chacun, indépendamment l'un de l'autre, H, Cl, un alkyle en $C_1$–$C_{12}$, un cycloalkyle en $C_5$–$C_8$, un phényle, un phénylalkyle en $C_7$–$C_9$, –OH ou un alcoxy en $C_1$–$C_{12}$,

$R^4$ et $R^5$ représentent chacun, indépendamment l'un de l'autre, H, Cl, –OH, un alcoxy en $C_1$–$C_{12}$ ou un alkyle en $C_1$–$C_{12}$,

$R^6$ représente H ou $-CH_3$,

$R^7$ représente H, O˙, un alkyle en $C_1$–$C_{12}$, un alcenyle en $C_3$–$C_8$, un benzyle, un acétyle ou un radical

$$-CH_2-CH(OH)-R^{13}$$

$R^8$ représente un alkyle en $C_1$–$C_{12}$, un alcényle en $C_3$–$C_8$ ou un phénylalkyle en $C_7$–$C_9$,

$R^9$ représente un radical

$-CH_2CH{=}CHCH_2-$ 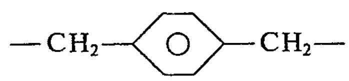

or

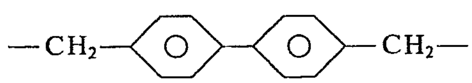

$R^{10}$ et $R^{11}$ représentent chacun l'hydrogène, un alkyle en $C_1$–$C_{12}$, un phényle ou un benzyle ou

$R^{10}$ et $R^{11}$ forment ensemble un alkylène en $C_4$–$C_{19}$,

$R^{12}$ représente H ou une valence libre,

$R^{13}$ représente H, $-CH_3$, $-C_2H_5$ ou un phényle,

X représente un radical

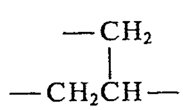

ou

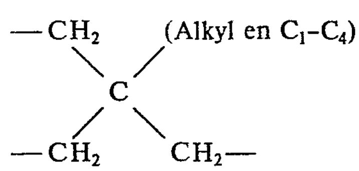

et

Y représente un alkylène en $C_2$–$C_8$,

les radicaux A étant reliés aux radicaux B par une liaison directe ou par un groupement bivalent Z formant pont, lequel Z peut être l'un des radicaux siuvants:

$-(CH_2)_m-CO-O-$ $\quad -(CH_2)_m-CO-O-R^{14}-$ $\quad -O-(CH_2)_n-CO-O-R^{14}-$

$-CH_2-CH(OH)-CH_2-O-CO-(CH_2)_m-$

$-O-CH_2-CH(OH)-CH_2-O-CO-(CH_2)_m-$

$-CH_2-CH(OH)-CH_2-$ $\quad -CH_2-CH(OH)-CH_2-O-$

$-CH_2-CH(OH)-CH_2-N(R^{15})-(CH_2)_m-$

$-O-CH_2-CH(OH)-CH_2-N(R^{15})-(CH_2)_m-$

$-N(R^{15})-CH_2-CH(OH)-CH_2-N(R^{16})-(CH_2)_m-$,

$-CH_2-CH(R^{13})-O-CH_2-CH(OH)-CH_2-N(R^{15})-(CH_2)_m-$

$-CH_2-CH(R^{13})-O-CH_2-CH(OH)-CH_2-O-CO-(CH_2)_m-$

$-(CH_2)_m-N(R^{15})-CO-$ $\quad -(CH_2)_m-N(R^{15})-CO-(CH_2)_n-$

$-O-(CH_2)_3-N(R^{15})-CO-(CH_2)_m-$ $\quad -CH_2-NH-CO-CH_2-O-$

$-CH_2-NH-CO-CH_2-O-CH(R^{13})-CH_2-$

$-CH_2-NH-CO-CH_2-N(R^{15})-(CH_2)_m-$

$-CH_2-NH-CO-CH_2CH_2-N(R^{15})-(CH_2)_m-$

et

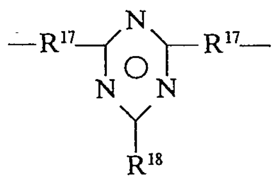

dans lequels m est égal à 0, à 1 ou à 2, n est égal à 1 ou à 2, $R^{14}$ représente $-CH_2-$ ou $-CH(R^{13})-CH_2-$, $R^{15}$ et $R^{16}$ représentent chacun H, un alkyle en $C_1-C_{12}$, un cyclohexyle ou un benzyle, $R^{17}$ représente $-O-$ ou $-NR^{15}-$ et $R^{18}$ représente un alcoxy en $C_1-C_{12}$, un phénoxy ou un groupe amino

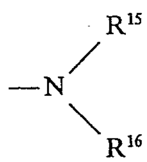

ou les radicaux A et B étant reliés par un radical trivalent répondant à la formule:

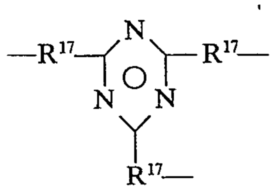

ou un radical A II étant relié à un radical B par un groupement $-O-(CH_2)_n-CO-O-$.

2. Composés selon la revendication 1 qui contiennent 1 ou 2 des radicaux B I à B VI.

3. Composés selon la revendication 2 dans lesquels $R^6$ représente l'hydrogène, $R^7$ l'hydrogène, un alkyle en $C_1-C_8$, un allyle ou un benzyle et $R^8$ un alkyle en $C_1-C_{12}$, un alcényle en $C_3-C_5$ ou un benzyle.

4. Composés selon la revendication 1 qui contiennent un radical de formule A I dans lequel $R^1$ représente l'hydrogène ou le chlore.

5. Composés selon la revendication 1 dans lesquels les radicaux A sont reliés aux radicaux B par une liaison directe ou par l'un des radicaux suivants: $-(CH_2)_m-CO-O-$, $-(CH_2)_m-CO-O-R^{14}-$, $-O-(CH_2)_n-CO-O-R^{14}-$, $-(CH_2)_m-N(R^{15})-CO-$, $-(CH_2)_m-N(R^{15})-CO-(CH_2)_n-$, $-CH_2-NH-CO-CH_2-O-$ et $-CH_2-NH-CO-CH_2-N(R^{15})-(CH_2)_m-$.

6. Composés selon la revendication 1 dans lesquels les radicaux A sont reliés aux radicaux B par un radical répondant à l'une des formules:

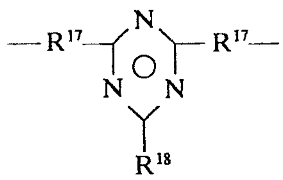 et 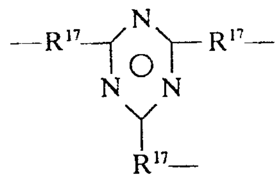

dans lesquelles $R^{17}$ représente un groupe $-NR^{15}-$, le symbole $R^{15}$ ayant la signification donnée à la revendication 1.

7. Application de composés selon la revendication 1 comme stabilisants pours des polymères organiques contre les dommages que peut occasionner la lumière.

8. Application selon la revendication 7, caractérisée en ce que le polymère organique est un feuillogène pour peintures ou vernis.

9. Polymère organique stabilisé contre les effets destructeurs de la lumière, polymère caractérisé en ce qu'il contient, comme stabilisant, de 0,05 à 5% en poids d'un composé selon la revendication 1.

10. Polymère stabilisé selon la revendication 9, caractérisé en ce que le polymère est un feuillogène pour peintures ou vernis.

11. Composés selon la revendication 1 dans lequels un radical A II est relié à un radical B par un groupement $-O-(CH_2)_n-CO-O-$.